# EUROPEAN PATENT APPLICATION

(11) **EP 2 316 434 A1**
(43) Date of publication of application: **04.05.2011**
(21) Application number: 09173725.4
(22) Date of filing: 22.10.2009
(51) Int. Cl.: A61K 9/20, A61K 31/13

(54) **Orally disintegrating tablets of memantine**

(71) Applicant: Abdi Ibrahim Ilac Sanayi Ve Ticaret Anonim Sirketi, 34555 Hadimkoy, Istanbul (TR)
(72) Inventor: Farshi, Farhad, 34555 ISTANBUL (TR); Kandemir, Levent, 34555 ISTANBUL (TR); Kaplan, Muge, 34555 ISTANBUL (TR); Dogan Esra, 34555 ISTANBUL (TR); Koc, Fikret, 34555 ISTANBUL (TR); Soylemez, Serdar, 34555 ISTANBUL (TR)

(57) **Abstract**

The present invention is related to orally disintegrating tablets of memantine characterized in that said tablets are obtained by direct compression method and memantine particles do not require coating in order to taste masking.

## Description

### Technical Field

The present invention is related to orally disintegrating tablets of memantine characterized in that said tablets are obtained by direct compression method and memantine particles do not require coating in order to taste masking .

### Background Art

In the patent of WO 2009/084017 (RUBICON RESEARCH PRIVATE LIMITED) 10.10.2007 , it is disclosed that coating of memantine particles with coating agents in order to obtain taste masked orally disintegrating tablets.

### Disclosure of Invention

The present invention provides a fast disintegrating dosage forms of memantine obtained by direct compression methods. Fast disintegrating dosage forms include an effective amount of memantine or a pharmaceutically acceptable salt thereof and at least one pharmaceutically acceptable excipient.

According to this invention, In fast disintegrating dosage forms, pharmaceutical formulations are disintegrated in less than three minutes in oral cavity. More preferably, the pharmaceutical formulations are disintegrated in an oral cavity in less than two minutes and most preferably the pharmaceutical formulations are disintegrated in oral cavity in less than one minute.

Fast-dissolving or fast-disintegrating tablets are useful for elderly people, children and patients who have difficulties swallowing tablets. Such peoples are unwilling and/or unable to swallow tablets, capsules or other traditional solid dosage forms. This is especially the case of pharmaceuticals for paediatric or geriatric use. Where patients who are not able to take it with water, fast disintegrating dosage forms would be beneficial . To solve this problem, tablets that disintegrate rapidly in the mouth were developed. Such tablets are rapidly disintegrated when contact with saliva in the oral cavity.

A fast-disintegrating tablets may have many numerous shapes, such as dish-like ,ellipsoid , rods, granules, blocks, cubes with rounded edges, or any other shape suitable for pharmaceutical administration.

Memantine hydrochloride is administered in pharmaceutically acceptable doses.

Direct compression agent is selected from the group consisting of pregelatinised starches, polyvinylpyrrolidone,methylcellulose, microcrystalline cellulose, sucrose, lactose, dextrose,sorbitol, mannitol, lactitol,xylitol, modified calcium salt, granulated corn starch, modified rice starch, compressible sugar,dextrate, dicalcium phosphate, hydroxypropylcellulose , methylcellulose , hydroxypropylmethylcellulose, polyethylene glycol, amylose,anhydrous calcium hydrogen phosphate, calcium sulphate,maltose, tribasic calcium phosphate, dibasic calcium phosphate, low-crystallinity powdered cellulose,silicified microcrystalline cellulose,chitin,chitosan hydrochloride, copovidone,croscarmellose sodium,dextrose, anhydrous lactose,anhydrous alpha lactose,anhydrous beta lactose,agglomerated lactose, spray-dried lactose, maltodextrin, co-processed anhydrous lactose- anhydrous lactitol, co-processed calcium sulphate-microcrystalline cellulose,fructose, co-processed lactose-cellulose,co-processed lactose-starch,co-processed lactose-povidone,coprecipitated sucrose-maltodextrin , carbohydrates such as Pharmaburst^{™} erythritol, isomalt, lacitol, maltitol, starch hydrolysate, polydextrose, glucose, mixtures thereof.

According to this invention, fast disintegrating or fast dissolving dosage form includes one or more pharmaceutically acceptable excipients, carriers, or diluents. In fast disintegrating or fast dissolving formulations, surfactants, diluents, sweeteners, disintegrants, binders, lubricants, glidants, colorants, flavors ,stabilizing agents mixtures thereof and the like can be used.

Acceptable excipients are, but not limited to, calcium sulfate, starch, mannitol, kaolin, sorbitol, xylitol, sodium chloride, sodium bicarbonate, citric acid, powdered cellulose derivatives, microcrystalline cellulose, pullulan, silicified microcrystalline cellulose, ammonium bicarbonate, carrageenan, carbohydrates such as Pharmaburst™, magnesium carbonate, tribasic calcium phosphate, calcium sulfate, magnesium oxide, poloxamer,gums, hydroxypropyl methylcellulose, gelatin, mixtures thereof, and the like .

Diluents are, but not limited to, mannitol, sorbitol, xylitol, microcrystalline cellulose, silicified microcrystalline cellulose , hydroxypropyl methylcellulose, hydroxypropyl cellulose , pullulan and fast dissolving carbohydrates such as Pharmaburst™, mixtures thereof and the like.

Glidants are, but not limited to, silicon dioxide, colloidal silicon dioxide, calcium silicate, magnesium silicate, magnesium trisilicate, talc, starch , mixtures thereof or whatsoever.

Binders are, but not limited to, sodium alginate,cellulose, methylcellulose, ethylcellulose, hydroxypropyl cellulose, hydroxypropyl methylcellulose, sodium carboxymethyl cellulose, polypropylpyrrolidone, polyvinylprrolidone, gelatin, polyethylene glycol, starch, pre-gelatinized starch, sugars, trehalose, glucose,tragacanth,sorbitol, acacia, alginates, carrageenan, xanthan gum, locust bean gum and gum arabic, waxes,polyacrylamide , mixtures thereof, and whatsoever.

Lubricants are, but not limited to, calcium stearate, glyceryl monostearate, glyceryl behenate, glyceryl palmitostearate, hydrogenated vegetable oil, light mineral oil, magnesium stearate, mineral oil, polyethylene glycol, poloxamer, sodium benzoate, sodium lauryl sulfate, sodium stearyl fumarate, stearic acid, talc, zinc stearate, mixtures thereof or whatsoever.

Disintegrants are, but not limited to, sodium starch glycolate, sodium carboxymethyl cellulose, calcium carboxymethyl cellulose, croscarmellose sodium, crospovidone, chitosan, agar, alginic acid, calcium alginate, methyl cellulose, microcrystalline cellulose, powdered cellulose, lower alkylsubstituted hydroxypropyl cellulose,hydroxylpropyl starch, low-substituted hydroxypropylcellulose, polacrilin potassium, starch, pregelatinized starch, sodium alginate, magnesium aluminum silicate, polacrilin potassium, povidone, sodium starch glycolate, mixtures thereof and the like.

Flavors are, but not limited to, cinnamon oil,essence of apple, essence of pear, essence of peach, essence of grape, essence of strawberry, essence of raspberry, essence of cherry,essence of plum, essence of pineapple, essence of apricot, oil of wintergreen, peppermint oils, clove oil, bay oil, anise oil, eucalyptus, thyme oil, cedar leaf oil, oil of nutmeg, oil of sage, oil of bitter almonds, cassia oil, citrus oils such as lemon, orange, grape, lime and grapefruit ,gurana, marshmallow, berries, vanilla, benzaldehyde , aldehyde C-8 , licorice, raspberry, aldehyde C-9, aldehyde C-12 , tolyl aldehyde ,bubble gum, sarsaparilla compound, miracle berry, aromatic elixir, extracts of gurana, glycyrrhiza elixir, mixtures thereof or whatsoever.

Sweeteners are but not limited to, corn syrup,dextrose, invert sugar, fructose, saccharin, aspartame, acesulfame-K, Stevia rebaudiana, sucralose, sorbitol, mannitol, zylitol, mixtures thereof and the like

Other ingredients such as colorants and titanium dioxide can also be used.

The fast-disintegrating or fast dissolving dosage form of memantine is a solid dosage form that does not require water for swallowing.

Fast disintegrating or fast dissolving dosage form is typically orally disintegrating tablet that dissolves or disperses rapidly when in contact with saliva.

Orally disintegrating tablet can comprise sugar-based excipients, super-disintegrating agents, effervescent agents and further suitable excipients .

Another embodiment of the present invention provides a carbohydrate-based fast dissolving dosage form. Co-processed carbohydrates can be used in embodiments.

This invention provides taste masked orally disintegrating tablets.

In fast dissolving or fast disintegrating dosage forms one or more taste masking excipients such as flavors, sweeteners, acidic amino acids, lipids, and surfactants can further be used.

An additional problem arising from oral administration .Orally disintegrating tablets contains a bitter or irritating drug which need to be masked in the oral cavity. Therefore a taste masking layer should be created. However in this invention taste masking layer is not required.

Memantine hydrochloride orally disintegrating tablet comprises; memantine hydrochloride is in the range of from about 1 % to about 30 %, binder is in the range of from about 0.1 % to about 40 %, direct compression agent is in the range of from about 10 % to about 90 %, disintegrant is in the range of from about 0.1 % to about 15 %, sweetener is in the range of from about 0.1 % to about 10 %, flavour is in the range of from about 0.1 % to about 15 %, glidant is in the range of from about 0.1 % to about 10 %, lubricant is in the range of from about 0.1 % to about 20 % by weight.

The most preferred orally disintegrating tablet of memantine hydrochloride comprises; memantine is about 4 %, binder is about 20 %, direct compression agent is about 64.90 %, disintegrant is about 4 %, sweetener is about 1 %, flavour is about 1.60 %, glidant is about 3 %, lubricant is about 1.50 % by weight.

This invention also provides a preparation method which has following steps :
1. Diluent, binder and glidant are loaded,
2. Sieved mixture of disintegrant, memantine hydrochloride, sweetener and flavour are added to mixture obtained from step 1,
3. Obtained powder mixture from step 2 is blended,
4. Sieved lubricant is added to the blended powder mixture obtained from step 3,
5. Final powder mixture, obtained from step 4, is blended ,
6. The prepared powder mixture is compressed

## Claims

1. A fast disintegrating or fast dissolving dosage form of memantine or pharmaceutically acceptable salts thereof **characterized in that** the bitter taste of memantine is not masked using coating techniques and dosage form is prepared by direct compression wherein direct compression agent is selected from the group consisting of pregelatinised starches, polyvinylpyrrolidone,methylcellulose, microcrystalline cellulose, sucrose, lactose, dextrose,sorbitol, mannitol, lactitol,xylitol, modified calcium salt , granulated corn starch, modified rice starch, compressible sugar,dextrate, dicalcium phosphate, hydroxypropylcellulose , methylcellulose , hydroxypropylmethylcellulose, polyethylene glycol, amylose,anhydrous calcium hydrogen phosphate, calcium sulphate,maltose, tribasic calcium phosphate, dibasic calcium phosphate, low-crystallinity powdered cellulose,silicified microcrystalline cellulose,chitin,chitosan hydrochloride, copovidone,croscarmellose sodium,dextrose, anhydrous lactose,anhydrous alpha lactose,anhydrous beta lactose,agglomerated lactose, spray-dried lactose, maltodextrin, co-processed anhydrous lactose- anhydrous lactitol, co-processed calcium sulphate-microcrystalline cellulose,fructose, co-processed lactose-cellulose,co-processed lactose-starch,co-processed lactose-povidone,coprecipitated sucrose-maltodextrin , carbohydrates such as Pharmaburst™ erythritol, isomalt, lacitol, maltitol, starch hydrolysate, polydextrose, glucose or mixtures thereof.

2. According to claim 1, the fast disintegrating or fast dissolving dosage form of memantine or its pharmaceutically acceptable salt is disintegrated in oral cavity in less than three minutes.

3. According to claim 2, the fast disintegrating or fast dissolving dosage dosage form of memantine or its pharmaceutically acceptable salt is disintegrated in oral cavity in less than two minutes.

4. According to claim 3, the fast disintegrating or fast dissolving dosage dosage form of memantine or its pharmaceutically acceptable salt is disintegrated in oral cavity in less than one minutes.

5. A preparation method of fast disintegrating or fast dissolving dosage form of memantine or pharmaceutically acceptable salts thereof **characterized in that** said method has the following steps : a. Diluent, binder and glidant are loaded, b. Sieved mixture of disintegrant, memantine, sweetener and flavour are added to mixture obtained from step a, c. Obtained powder mixture from step b is blended, d. Sieved lubricant is added to the blended powder mixture obtained from step c, e. Final powder mixture, obtained from step d, is blended f. The prepared powder mixture is compressed.

6. A fast disintegrating or fast dissolving dosage form of memantine or pharmaceutically acceptable salts thereof comprising; memantine or its pharmaceutically acceptable salt is in the range of from 1 % to 30 %, binder is in the range of from 0.1 % to 40 %, direct compression agent is in the range of from 10 % to 90 %, disintegrant is in the range of from 0.1 % to 15 %, sweetener is in the range of from 0.1 % to 10 %, flavour is in the range of from 0.1 % to 15 %, glidant is in the range of from 0.1 % to 10 %, lubricant is in the range of from 0.1 % to 20 % by weight.

7. According to claim 6, fast disintegrating or fast dissolving dosage form of memantine or pharmaceutically acceptable salts thereof comprising; memantine is 4 %, binder is 20 %, direct compression agent is 64.90 %, disintegrant is 4 %, sweetener is 1 %, flavour is 1.60 %, glidant is 3 %, lubricant is 1.50 % by weight.

8. According to preceding claims, the fast disintegrating or fast dissolving dosage form is orally disintegrating tablet.
